Europäisches Patentamt

**(19)** European Patent Office

Office européen des brevets

**(11)** Publication number : **0 439 013 B1**

# **(12)** EUROPEAN PATENT SPECIFICATION

**(45)** Date of publication of patent specification :
**24.11.93 Bulletin 93/47**

**(51)** Int. Cl.$^5$ : **B01J 10/00,** B01J 19/18,
C07C 51/235, C07C 53/128

**(21)** Application number : **91100124.6**

**(22)** Date of filing : **03.01.91**

**(54)** Enhanced gas-liquid reactions.

**(30)** Priority : **04.01.90 US 460815**

**(43)** Date of publication of application :
**31.07.91 Bulletin 91/31**

**(45)** Publication of the grant of the patent :
**24.11.93 Bulletin 93/47**

**(84)** Designated Contracting States :
**BE DE ES FR GB IT NL**

**(56)** References cited :
**EP-A- 0 374 967
GB-A- 782 430
US-A- 3 361 533
CHEMICAL ENGINEERING PROGRESS, vol.
81, no. 5, November 1985, pages 36-39, Aiche,
New York, US; L.M. LITZ: "A novel gas-liquid
stirred tank reactor"**

**(73)** Proprietor : **PRAXAIR TECHNOLOGY, INC.
39 Old Ridgebury Road
Danbury, CT 06810-5113 (US)**

**(72)** Inventor : **Weise, Mark Kurt
46 N. 17th Street
Prospect Park, New Jersey 07508 (US)**
Inventor : **Adis, Mitchell
39 Laurence Drive
North White Plains, New York 10603 (US)**

**(74)** Representative : **Schwan, Gerhard, Dipl.-Ing.
Elfenstrasse 32
D-81739 München (DE)**

## Description

Field of the Invention -

The invention relates to the reaction of gases and liquids. More particularly, it relates to the enhancing of such reactions.

Description of the Prior Art -

A wide variety of techniques and systems are known for achieving the mixing and reaction of gases and liquids. Bubble column reactors are one simple type of gas-liquid reaction system. In such a system, the gas is injected, in the form of gas bubbles, at the bottom of a vessel containing a body of liquid. The gas bubbles rise upwardly through the body of liquid to achieve the desired mixing and reaction. In other systems, mechanical agitation means are often employed to improve such gas-liquid mixing and reaction. Thus, surface aerators, jet and stirred tank containing impellers have been used for this purpose.

In a process known from GB-A-782 430 2-ethylhexaldehyde is oxidized by continuously adding such aldehyde and an aqueous sodium hydroxide solution to a charge of water and of silver catalyst in a reaction vessel equipped with a motor-driven, propeller-type stirrer, whilst maintaining the reaction temperature between 20 °C and 30 °C. The filtered product solution of sodium 2-ehtylhexanoate is steam destilled to remove volatile impurities and then acidified with a slight excess of sulfuric acid. The lower aqueous layer is steam-distilled, and the upper layer of the acidified solution is combined with the acid recovered by the steam distillation of the aqueous layer. The crude acid obtained thereby finally is refined by vacuum distillation to yield refined 2-ethylhexanoic acid.

Furthermore it is known from US-A-3 361 533 to produce hydrogen peroxide by contacting hydrogen and oxygen with a solid catalyst in the liquid phase in the presence of water, an acid at least as strong as acidic acid, and a non-acetic oxygen-containing organic compound selected from the group consisting of alcohols, aldehydes, ketones, ethers, amides and oxygene-containing amines. In one example hydrogen peroxide preparations were carried out at 10°C, 0 °C and - 10 °C by passing a mixture of hydrogen with air into a solution in 75 % acetone + 25 % water (by volume) of sulphuric acid and hydrochloric acid in which was suspended a finely divided catalyst consisting of 5 % palladium supported on silica gel. It was observed that increasing yields were obtained at decreasing temperature.

Stirred tank reactors (STR) are commonly used for gas-liquid mixing and reaction, either on a batch or a continuous basis, in applications having residence times of more than a few minutes. Litz, in "A Mixed Gas-Liquid Stirred Tank Reactor", CEP, November, 1985, pp. 36 - 39, discloses that gas is normally fed to a sparger at the bottom of a conventional STR, and a flat-bladed Rushton turbine or other such mixer system is used to shear the gas into small bubbles for dispersion in the liquid phase. Axial flow impellers are commonly employed to facilitate gas dissolution. Further dissolution occurs as the gas bubbles rise up through the liquid. Undissolved gas that reaches the gas-liquid interface in the upper part of the STR is normally lost.

The Litz publication refers to a new design STR, called an Advance Gas Reactor (AGR), that enables gas dissolution and chemical reaction rates to be increased, while gas and power consumption is being reduced. In this AGR system, a down-pumping impeller is employed within a hollow draft tube, with a baffle arrangement at the upper, inlet thereof causing the liquid flowing into the top of the draft tube to form vortices. These vortices cause feed gas from the overhead gas phase above the liquid to pass down through the draft tube along with the recirculating liquid, as described in the Roeckel et al. patent, U.S. 4,328,175 and the Litz patent, U.S. 4,454,077, and its reissue patent, Re. 32,562.

With respect to various organic compounds that can burn or explode when their vapor is admixed with an oxygen-rich gas, a further development has been made and patented to avoid reliance on vortex characteristics to assure against the creation of a potentially explosive vapor-oxygen mixture in the overhead gas space. This development is referred to as a Liquid Organic Reactor (LOR) system, which provides for impeller means to be positioned in a portion of the body of liquid to create a recirculating flow pattern, with air, oxygen or oxygen enriched air being introduced directly into said recirculating liquid. This recirculating portion of liquid is separated from, but in fluid communication with, a quiescent portion of said body of liquid having a gas-liquid interface, with an overhead gas space in said reaction vessel. The recirculation flow pattern and rate are such as to effectively preclude the passage of gas through the quiescent liquid and into the overhead space.

While the mixing and reaction of oxygen with organic liquids can be enhanced by the use of STR, AGR and LOR systems in particular applications, there remains a need for further improvements in the process of reacting organic liquids with oxygen. Thus, greater efficiency of such reactions, as measured by reaction rates, product yields and the like, is generally desired in the art to satisfy the ever-increasing technical and economic reuqirements of present-day industrial operations.

It is an object of the invention, therefore, to provide an improved process for the reaction between organic liquids and oxygen.

It is another object of the invention to provide a process for enhancing the efficiency of the reaction of organic liquids and oxygen.

With these and other objects in mind, the invention is hereinafter described in detail, the novel features thereof being particularly pointed out in the appended claims.

## SUMMARY OF THE INVENTION

The subject invention provides for a gas-liquid process for the oxidation of an organic chemical the oxidation of which involves free radical mechanisms and the formation of reaction intermediates that tend to decompose at conventional reaction temperatures or undergo undesired side reactions, comprising:

(a) introducing air, oxygen or oxygen-enriched air into a body of liquid containing said organic chemical in a mechanically stirred type reaction system in which impeller means are employed to impart shear energy to said liquid and achieve bulk mixing thereof; and

(b) maintaining the stirred type reaction system at a reaction temperature down to about - 10 °C or below.

The reaction rate and/or product yield for reactions between oxygen and an organic liquid are substantially increased by decreasing the reaction temperature in stirred tank reactors as compared to that employed in non-stirred reaction vessels.

The objects of the invention are accomplished by the discovery of an unexpected interaction between reaction temperature and the type of reaction system employed. Thus, an advantageous combination of lower reaction temperature and a mechanically stirred tank type reactor system, such as STR, AGR or LOR, unexpectedly enables the reaction rate and product yield to be increased.

It is generally accepted that chemical reactions proceed more rapidly as the reaction temperature is increased. The well known Arrhenius equation relates the reaction rate constant to temperature, showing an exponential increase in reaction rate with an increase in temperature. A common rule of thumb is that for every 10°K increase in temperature, there is a doubling of reaction rate for simple reactions. More complex reactions will depart from this generalization, but will almost always show an increase in reaction rate with an increase in temperature. The process design of reactor systems, therefore, is usually based on operation at the highest possible reaction temperature consistent with pertinent safety, economy and product characteristic factors.

The gas-liquid mass transfer characteristics of a mechanically stirred tank reactor are generally better than those of a non-mechanically stirred bubbling type reactor. This results from the shear energy input of the mixing impeller employed and the resultant bulk mixing of the liquid. Higher mass transfer rates mean that the reactant gas, such as oxygen, dissolves in the liquid reactant at a faster rate than in non-mechanically stirred reactors. The gas must typically be dissolved in the liquid for the reaction to take place. The faster the gas dissolves, the faster can be the rate of reaction. A mechanically stirred reactor will also generally have a high gas utilization efficiency, which is a very important factor in operations using a relatively expensive pure gas, such as pure oxygen. The LOR system, in particular, provides very good liquid-liquid mixing, which may strongly influence the overall reaction path.

For more complicated, less understood reasons, lowering the reaction temperature has been found to result in an increase in yield or reaction efficiency, particularly in the case of operations involving reaction intermediates such as free radicals. Such intermediates are more reactive than the reactants themselves, and may have a tendency to decompose or undergo side reactions before having a chance to form the desired product. An increase in temperature may increase the rate of decomposition of such reaction intermediates, or their side reaction, in some systems. These effects may be amplified in the high mass transfer environment of a stirred tank reactor. Such undesired results are circumvented by the discovery of the advantageous use of lower reaction temperatures in stirred tank reactors in the practice of the invention.

The advantageous and unexpected benefits of the combination of a mechanically stirred reactor system and lower operating temperature were discovered in the course of a program to expand the scope of gas-liquid reactions processed in such stirred type reactor systems. Stirred reactor systems, e.g., AGR systems, had been successfully employed in laboratory, pilot plant and commercial scale operations, batch and continuous, for aqueous oxidations and organic chemical hydrogenations, and it was desired to expand their use, e.g. in AGR or LOR systems, to include the oxidation of organic chemicals.

A known organic oxidation process carried out in commercial operations is the oxidation of 2-ethylhexaldehyde to 2-ethylhexanoic acid. This process is commonly carried out by sparging air at 929 kPa (120 psig)to supply the necessary oxygen for this reaction in a bubble column type reactor. The reaction rate, expressed in millimoles of oxygen per liter (mmols/l/min.) of liquid per minute, is nominally about 104 in the first of three

3

reactors in series. Pure oxygen was compared to air in a 3 liter LOR laboratory reactor at comparable 80°C temperature and atmospheric pressure. No increase in the reaction rate was found for pure oxygen compared to air in such laboratory tests. However, the reaction rate using either oxygen or air in the laboratory was found to be 8-14 mmols/l/min., very appreciably less than achieved in commercial bubble column practice. Although the oxygen partial pressure was lower in the laboratory runs, this did not account for this drastic difference in reaction rate.

In addition, gas chromatographic analysis of the product from the laboratory tests showed that the yield of product acid was lower than in said commercial practice. Whereas the product yield in commercial practice was about 85%, with close to 100% conversion, the yield obtained in laboratory test was about 60%, based on the stoichiometric amount, at the same conversion. Furthermore, in addition to forming the desired eight carbon 2-ethylhexanoic acid product, the test reaction also undesirably formed substantial quantities of seven-carbon products, such as n-heptane, n-heptanal and n-heptanol. This will be understood to require the splitting off of one carbon atom during the reaction process.

In light of such results, a 25 mm (1 inch) diameter by 305 mm (12 inch) long glass bubble column with a fritted disk was used as a convenient reactor to test the reaction system without the large volume required in a stirred reactor. Tests carried out using pure oxygen at the same temperature and pressure as in the 3-liter apparatus gave reaction rates of about 25 mmols/l/min., i.e. 2.5 times greater than in the stirred reactor. As contamination in the stirred reaction vessel was initially suspected as the cause for the difference in result, various possible contaminants from the stirred reactor or the reactant storage vessel were added to the bubbler vessel to determine their effect on the reaction rate. No significant change was caused thereby. Since the commercial bubble column system was a low shear reactor, the 3-liter LOR laboratory system was operated at lower shear by removal of the lower flat blade turbine impeller normally employed below the helix of the down pumping impeller in the LOR system. Tests using this configuration, however, produced no difference in result. Various other changes, such as reducing the impeller speed, increasing the impeller speed, changing reactant concentrations or eliminating the conventional reaction catalyst, were tried but found to produce no improvement in result.

During the course of such comparative tests, however, it was noted that, at some point in the laboratory tests, the reaction temperature in the reaction vessel had dropped from 80°C to about 60°C. Significantly, the oxygen content in the overhead gas space dropped from 1.5% to about 0.5% by volume. This indicated that the oxygen fed to the reaction vessel was being consumed at a greater rate than at the conventional 80°C temperature.

In a subsequent test, 2.5 liters of 100% 2-ethylhexaldehyde were charged to the laboratory LOR vessel for oxidation with pure oxygen at atmospheric pressure. No heat was added to the system. The reaction with oxygen began at ambient temperature, i.e., 76°F (about 24.5°C). The reaction rate during the batch reaction averaged 33.3, and peaked at 47.7, mmols/l/min. Cooling water was used to control the reaction temperature to below 115°F (46°C) for the first hour. The batch was then cooled to 96°F (35.5°C) and the rate remained the same. A series of such lower temperature batch runs of the subject reaction were thereafter carried out and are summarized in Table 1 as follows:

## TABLE 1

### BATCH OXIDATIONS

| Run | Temp (°C) | Reaction Rate (mmols/l/min) | Reactor Type | Product Yield (%) |
|-----|-----------|------------------------------|--------------|-------------------|
| 1 | 60 | 3.16 | LOR | – |
| 2 | 60 | 2.81 | LOR | – |
| 3 | 60 | 1.77 | LOR | – |
| 4 | 60 | 2.75 | LOR | – |
| 5 | 60 | 1.26 | LOR | – |
| 6 | 60 | 2.76 | LOR | – |
| 7 | 80 | 10.87 | LOR | – |
| 8 | 80 | 34.73 | BUBBLER | – |
| 9 | 80 | 26.12 | BUBBLER | – |
| 10 | 80 | 23.73 | BUBBLER | – |
| 11 | 80 | 32.50 | BUBBLER | – |
| 12 | 80 | 29.03 | BUBBLER | – |
| 13 | 80 | 26.93 | BUBBLER | – |
| 14 | 80 | 25.63 | BUBBLER | – |
| 15 | 80 | 9.31 | LOR | – |
| 16 | 80 | 10.43 | LOR | – |
| 17 | 80 | 10.87 | LOR | 59.2 |
| 18 | 80 | 9.68 | LOR | 64.1 |
| 19 | 80 | 10.86 | LOR | – |
| 20 | 80 | 7.44 | LOR | – |
| 21 | 80 | 7.83 | LOR | – |
| 22 | 80 | 6.98 | LOR | – |
| 23 | 40 | 33.33 | LOR | 81.3 |
| 24 | 45 | 19.39 | LOR | 96.9 |
| 25 | 55 | 23.50 | LOR | – |
| 26 | 50 | 26.28 | LOR | 96.0 |
| 27 | 55 | 36.60 | LOR | 88.1 |
| 28 | 40 | 22.49 | LOR | 93.6 |

As will be seen, the product yield or selectivity increased significantly from around 60% at 80°C to as high as 96% at the lower temperatures indicated. During such batch runs, some experimentation was done, such as increasing and decreasing the temperature as the batch proceeded to observe the effect thereof upon the reaction rate. Once it was established that higher rates and product yield or selectivity were achieved at lower temperatures, the laboratory apparatus was set up to run as a continuous, stirred tank type system.

Five continuous runs were carried out in this system, simulating the 85% plant yield at 100% conversion achieved in the first reactor in commercial practice. A reaction rate of 40 mmol/l/min. and a 96% acid product

5

yield at 100% conversion were obtained at 40°C and 46°C. When the bubbler type system was employed in the commercial practice, the reaction rate was 104 mmols/l/min. at an acid product yield of 92% at 100% conversion.

It was then determined that the reaction rate can be increased by an increase in pressure, without decreasing product yield, by operating at lower temperature levels. For this purpose, a 3.8 liter (one gallon) autoclave LOR system was employed. The results of continuous and batch runs therein were as set forth in Table 2 below.

EP 0 439 013 B1

## TABLE 2
### 2-ETHYLHEXALDEHYDE OXIDATION IN 3.8 LITER (ONE GALLON) AUTOCLAVE LOR SYSTEM

| PRESSURE | | TEMPERATURE | | AGITATION | OXYGEN IN VENT | % OXYGEN REACTED | REACTION RATE | PRODUCT YIELD |
|---|---|---|---|---|---|---|---|---|
| (kPa) | (psig) | (°C) | (°F) | (rpm) | (%) | | mmols/$l$/min | (%) |
| **I.** CONTINOUS DATA | | | | | | | | |
| 205 | 15 | 36.7 | 98 | 1,000 | 0.20 | 98.98 | 61 | 99.2 |
| 205 | 15 | 44.4 | 112 | 1,000 | 0.20 | 98.40 | 38 | 98.5 |
| 205 | 15 | 46.7 | 116 | 1,000 | 0.17 | 98.78 | 44 | -- |
| 205 | 15 | 47.2 | 117 | 1,000 | 0.20 | 98.34 | 95 | 99.0 |
| 205 | 15 | 60.6 | 141 | 1,000 | 0.20 | 92.58 | 8 | 98.8 |
| 308 | 30 | 44.4 | 112 | 1,000 | 0.10 | 99.70 | 62 | 100.0 |
| 308 | 30 | 46.1 | 115 | 1,000 | 0.20 | 99.61 | 98 | 98.9 |
| 308 | 30 | 52.8 | 127 | 1,000 | 0.10 | 98.52 | 13 | 98.3 |
| 377 | 40 | 41.1 | 106 | 1,000 | 0.10 | 99.71 | 107 | -- |
| 377 | 40 | 43.3 | 110 | 1,000 | 0.10 | 99.61 | 80 | -- |
| 377 | 40 | 54.4 | 130 | 1,000 | 0.06 | 97.81 | 8 | -- |
| 329 | 33 | 47.2 | 117 | 1,200 | 0.04 | 99.82 | 117 | -- |
| 343 | 35 | 42.2 | 108 | 1,200 | 0.08 | 99.85 | 154 | -- |
| 343 | 35 | 45.0 | 113 | 1,200 | 0.09 | 99.85 | 181 | -- |
| 343 | 35 | 45.0 | 113 | 1,200 | 0.08 | 99.86 | 77 | -- |
| 377 | 40 | 46.1 | 115 | 1,200 | 0.15 | 99.83 | 220 | -- |
| 377 | 40 | 47.2 | 117 | 1,200 | 0.36 | 99.65 | 194 | -- |
| 377 | 40 | 54.4 | 130 | 1,200 | 0.21 | 99.80 | 262 | -- |
| 377 | 40 | 56.1 | 133 | 1,200 | 0.21 | 99.81 | 278 | -- |
| **II.** BATCH DATA | | | | | | | | |
| 115 | 2.0 | 46.1 | 115 | 1,000 | 1.50 | 92.92 | 55 | 96.57 |
| 119 | 2.5 | 47.2 | 117 | 1,000 | 4.10 | 84.45 | 62 | 96.57 |
| 308 | 30.0 | 45.0 | 113 | 1,000 | 0.47 | 99.11 | 54 | 94.73 |
| 322 | 32.0 | 47.8 | 118 | 1,000 | 0.10 | 99.50 | 111 | 96.71 |
| 377 | 40.0 | 45.6 | 114 | 1,000 | 0.15 | 99.96 | 172 | 96.25 |
| **III.** PLANT DATA | | | | | | | | |
| 929 | 120 | 76.7 | 170 | N/A | -- | -- | 104 | 92.00 |

It will be appreciated from the results above that the use of a mechanically stirred tank type reactor, together with reaction temperatures significantly lower than those pertaining to non-mechanically stirred reactors, e.g. commercial bubble column systems, enables desirably higher reaction rates and product yields to be obtained. Thus, reaction rates of up to 278 mmols/l/min. were obtained, with yields of 2-ethylhexanoic acid product of up to 100%.

The enhanced reaction rate and/or product yield obtainable in the practice of the invention by maintaining the reaction system at a reaction temperature down to - 10 °C or below will vary depending upon the particular gas-liquid reaction being carried out, the particular mechanically stirred tank type reactor actually employed, the concentration levels of reactants, the reaction pressure employed, and the degree to which the reaction temperature is lowered below that employed in comparable non-stirred type operations to achieve a particular reaction rate and/or product yield. While the unexpected benefits of the invention were discovered initially with respect to the oxidation of 2-ethylhexaldehyde to 2-ethylhexanoic acid by air or oxygen, it has general application to the oxidation of various organic chemicals, mainly to reactions involving free radical mechanisms and the formation of reaction intermediates that may tend to decompose at conventional reaction temperatures or undergo undesired side reactions. The invention can also apply to other classes of reaction involving a gas and a liquid, where side reactions may or may not be a problem. It will also be understood that desired oxidation reactions can be carried out using air, pure oxygen or any desired oxygen enriched air composition.

While the decrease in reaction temperature employed in any particular embodiment of the invention will vary depending upon the overall conditions pertaining to the gas-liquid reaction in question, the reaction temperature employed should be significantly less than the temperature that would otherwise be employed in comparable non-stirred type reactor systems. Indeed, as will be appreciated from the above, such a significant reduction in reaction temperature may actually be required not only to achieve the unexpected benefits of higher reaction rates and/or increased product yields, but to avoid obtaining lower rates and yields than in non-stirred type reactor systems. For purposes of the invention, a reaction temperature decrease of at least about 20°C as compared with non-stirred tank reactors should be employed, with a decrease of about 40°C or more being generally preferred. When cooling water is employed to achieve such reaction temperature reduction, it is generally possible to reduce the temperature down to about 10°C. A further decrease in reaction temperatures of down to the reactive temperature of about -10°C or below as employed in conformity with the subject invention can be reached by the use of other known cooling means.

While it is within the scope of the invention to employ any stirred tank reactor system in which shear energy is imparted by the particular mixing impeller means employed and bulk mixing of the liquid is achieved, it is generally preferred to employ relatively high shear energy systems with a substantial volume at high turbulence levels, such as the AGR and LOR systems described above. The particular stirred reactor system can be determined for any particular application, of course, upon consideration of the particular gas-liquid reaction involved and the level of performance necessary or desired in that application.

The discovery of the invention, wherein a lower, rather than a higher, operating temperature can be employed to achieve enhanced reaction rates and/or higher yields represents a particularly desirable advance in the art. Not only are lower temperature operations desirable from an economic and other operational point of view, it will be appreciated that undesirable intermediate product decomposition and/or side reaction can often be avoided by the use of lower reaction temperatures. The invention enables highly desirable, high shear, gas-liquid mixing systems to be applied more economically and effectively to a variety of organic chemical oxidation and other commercially important gas-liquid reaction operations.

## Claims

1. A gas-liquid process for the oxidation of an organic chemical the oxidation of which involves free radical mechanisms and the formation of reaction intermediates that tend to decompose at conventional reaction temperatures or undergo undesired side reactions, comprising:
   (a) introducing air, oxygen or oxygen-enriched air into a body of liquid containing said organic chemical in a mechanically stirred type reaction system in which impeller means are employed to impart shear energy to said liquid and achieve bulk mixing thereof; and
   (b) carrying out the stirred type reaction so that a temperature of about - 10 °C or below is reached.

2. The process of claim 1 in which said reaction system comprises a stirred tank reactor having impeller means therein.

3. The process of claim 1 in which said reaction system comprises a down-pumping impeller positioned within

EP 0 439 013 B1

a hollow draft tube, said system being adapted to cause the body of liquid to move downwardly in the draft tube and upwardly outside of said draft tube in a recirculating flow pattern, with the flow of liquid into the top of the draft tube forming vortices that draw air or oxygen from the overhead gas space in the reactor system.

4. The process of claim 1 in which said reactor system comprises impeller means positioned in a portion of the body of liquid to create a recirculating flow pattern, with air, oxygen or oxygen-enriched air being introduced directly into said recirculating liquid, which is separated from, but in fluid communication with, a quiescent portion of said body of liquid having a gas-liquid interface, with an overhead gas space in said reaction system, the recirculating flow pattern and rate being such as to effectively preclude the passage of gas through the quiescent portion of liquid and into the overhead gas space.

5. The process of any one of claims 1 to 4 in which the organic chemical being oxidized is 2-ehtylhexaldehyde and the reaction product is 2-ehtylhexanoic acid.

6. The process of any one of the preceding claims in which the introduced gas is oxygen.


**Patentansprüche**

1. Gas-Flüssigkeits-Verfahren für die Oxidation einer organischen Chemikalie, bei deren Oxidation freie radikalische Mechanismen und die Bildung von Reaktionszwischenprodukten beteiligt sind, die zur Zersetzung bei konventionellen Reaktionstemperaturen neigen oder unerwünschte Nebenreaktionen erleiden, bei dem
   (a) Luft, Sauerstoff oder mit Sauerstoff angereicherte Luft in eine die organische Chemikalie enthaltende Flüssigkeitsmasse in einem mit mechanischem Rühren arbeitenden Reaktionssystem eingebracht werden, bei dem eine Laufradanordnung verwendet wird, um die Flüssigkeit mit Scherenergie zu beaufschlagen und ein Vermischen der Flüssigkeitsmasse zu bewirken; und
   (b) die unter Rühren erfolgende Reaktion so ausgeführt wird, daß eine Temperatur von etwa -10°C oder darunter erreicht wird.

2. Verfahren nach Anspruch 1, bei dem das Reaktionssystem einen Rührtankreaktor mit einer darin befindlichen Laufradanordnung aufweist.

3. Verfahren nach Anspruch 1, bei dem das Reaktionssystem ein nach unten pumpendes Laufrad aufweist, das in einem hohlen Führungsrohr sitzt, wobei das System in der Lage ist, die Flüssigkeitsmasse zu einer Abwärtsbewegung in dem Führungsrohr und zu einer Aufwärtsbewegung außerhalb des Führungsrohres in einer umlaufenden Strömungsverteilung zu veranlassen, und wobei das Einströmen von Flüssigkeit in das obere Ende des Führungsrohres Wirbel bildet, die Luft oder Sauerstoff aus dem Kopfraum in dem Reaktorsystem mitziehen.

4. Verfahren nach Anspruch 1, bei dem das Reaktorsystem eine Laufradanordnung aufweist, die in einem Teil der Flüssigkeitsmasse angeordnet ist, um für eine umlaufende Strömungsverteilung zu sorgen, wobei Luft, Sauerstoff oder mit Sauerstoff angereicherte Luft in die umgewälzte Flüssigkeit unmittelbar eingeleitet werden, die von einem eine Gas/Flüssigkeits-Grenzfläche mit einem Kopfraum in dem Reaktorsystem aufweisenden ruhigen Teil der Masse getrennt ist, mit diesem aber in Strömungsverbindung steht, wobei die Verteilung und Rate der umlaufenden Strömung so beschaffen sind, daß der Durchtritt von Gas durch den ruhigen Teil der Flüssigkeit und in den Kopfraum effektiv ausgeschlossen wird.

5. Verfahren nach einem der Ansprüche 1 bis 4, bei dem die zu oxidierende organische Chemikalie 2-Ethylhexaldehyd ist und das Reaktionsprodukt 2-Ethylhexansäure ist.

6. Verfahren nach einem der vorhergehenden Ansprüche, bei dem das eingebrachte Gas Sauerstoff ist.


**Revendications**

1. Procédé gaz-liquide pour l'oxydation d'un composé chimique organique dont l'oxydation fait intervenir des mécanismes radicalaires et la formation d'intermédiaires réactionnels qui tendent à se décomposer aux

**EP 0 439 013 B1**

températures classiques de réaction ou à subir des réactions secondaires indésirables, comprenant :

(a) l'introduction d'air, d'oxygène ou d'air enrichi en oxygène dans une masse de liquide contenant ledit composé chimique organique dans un dispositif réactionnel à agitation mécanique dans lequel des pales d'agitation sont utilisées pour communiquer une énergie de cisaillement audit liquide et parvenir à un mélange de la masse de ce liquide ; et

(b) la conduite de la réaction sous agitation de telle sorte qu'une température égale ou inférieure à environ -10°C soit atteinte.

2. Procédé suivant la revendication 1, dans lequel le dispositif réactionnel comprend un réacteur à réservoir sous agitation renfermant un moyen d'agitation par pales.

3. Procédé suivant la revendication 1, dans lequel le dispositif réactionnel comprend un agitateur à pales à pompage descendant positionné à l'intérieur d'un tube creux d'aspiration, ledit dispositif étant apte à provoquer le déplacement de la masse de liquide vers le bas dans le tube d'aspiration et vers le haut à l'extérieur dudit tube d'aspiration suivant un schéma d'écoulement à recirculation, le courant de liquide dans la partie supérieure du tube d'aspiration formant des tourbillons qui aspirent l'air ou l'oxygène à partir de l'espace gazeux de tête dans le réacteur.

4. Procédé suivant la revendication 1, dans lequel le réacteur comprend un moyen d'agitation par pales positionné dans une partie de la masse de liquide pour créer un schéma d'écoulement à recirculation, de l'air, de l'oxygène ou de l'air enrichi en oxygène étant introduit directement dans ledit liquide en recirculation qui est séparé de, mais en communication de fluide avec, une partie quiescente de ladite masse de liquide comprenant une interface gaz-liquide, avec un espace gazeux de tête dans ledit réacteur, le schéma et la vitesse d'écoulement à recirculation étant tels qu'ils empêchent efficacement le passage de gaz à travers la portion quiescente de liquide et dans l'espace gazeux de tête.

5. Procédé suivant l'une quelconque des revendications 1 à 4, dans lequel le composé chimique organique soumis à l'oxydation est le 2-éthylhexaldéhyde et le produit de réaction est l'acide 2-éthylhexanoïque.

6. Procédé suivant l'une quelconque des revendications précédentes, dans lequel le gaz introduit est l'oxygène.